Europäisches Patentamt

European Patent Office

Office européen des brevets

(19)

(11) Publication number : **0 523 883 A1**

# EUROPEAN PATENT APPLICATION

(12)

(21) Application number : **92306078.4**

(22) Date of filing : **01.07.92**

(51) Int. Cl.$^5$ : **C02F 3/32, C02F 9/00**

(30) Priority : **01.07.91 ZA 915069**
**01.07.91 ZA 915070**
**01.07.91 ZA 915071**
**08.06.92 ZA 924159**

(43) Date of publication of application :
**20.01.93 Bulletin 93/03**

(84) Designated Contracting States :
**AT BE DE ES FR GB GR IT NL PT**

(71) Applicant : **WATER RESEARCH COMMISSION**
**Watko Building 491, 18th Avenue**
**Rietfontein, Pretoria Transvaal Province (ZA)**

(72) Inventor : **Rose, Peter Dale**
**11 Willshire Crescent**
**Grahamstown, Cape Province (ZA)**
Inventor : **Cowan, Ashton Keith**
**6 Robinson Street**
**Grahamstown, Cape Province (ZA)**

(74) Representative : **Clifford, Frederick Alan et al**
**MARKS & CLERK 57/60 Lincoln's Inn Fields**
**London WC2A 3LS (GB)**

(54) **A process for the treatment of saline effluents.**

(57)   The invention concerns a process for the treatment of effluent by growing algae in a growth stage under aerobic conditions in a saline medium, treating a saline effluent containing organic degradable material with the algae in the presence of bacteria capable of digesting at least some of the organic material and separating the liquid from the solid material. The algae may belong to genus Dunaliella of Spirulina. The effluent may be a tannery effluent, or an organic sewage effluent, particularly secondary sewage sludge. Addition of a flocculant followed by filtration may be involved.

EP 0 523 883 A1

THIS INVENTION relates to a process for the treatment of saline effluents and the disposal of organic wastes. The invention further provides a process which not only enables saline effluents to be treated but provides a useful function for these wastes, with the recovery of products from the treatment.

The Applicant is aware of known processes comprising algal high rate oxidation ponding for treating fresh water effluents. While these processes remove organics present in these effluents, the biomass product mostly consists of a mixture of algal species, bacteria and protozoa (sometimes termed albazod) and hence has little or no commercial value. Fresh water effluents usually do not provide a sufficient selection factor for predictably manipulating the predominance of a single algal species from which a commercial product(s) can reliably be recovered.

Furthermore, a serious problem with effluents in many countries is the increasing saline content of water obtained therefrom, whether it is used as fresh water for human consumption or for agricultural purposes, eg. for irrigation. The deterioration of public water systems due to increasing salinity is becoming a serious problem for South Africa and many other countries.

Solutions which have been recommended for dealing with this problem include the creation of salt water sinks or inland lakes, the disposal of such water on solid waste sites, mine tailing dams and ash dams at power stations, the injection of such water into deep wells and disused mines, particularly gold mines, the piping of such water to the coast and discharge into the ocean, solar evaporation ponding of such water and the recovery of soda ash therefrom, and the piping of such water to solar brine ponds for electricity production.

All of the above methods, while providing a temporary solution to the storage or removal of saline water, do not make good use of the water during its disposal and do not deal with environmentally offensive aspects. It would be desirable to provide a process which can enable this water, when it contains organics or heavy metal components, to be treated in an inexpensive manner, preferably avoiding an energy intensive high technology solution, while also avoiding permanent environmental damage which would be problematic for future generations.

With a view, at least partially, to meeting some of the above problems, the present invention provides a process for the utilization and/or the purification of a saline effluent to produce a water which should be more environmentally acceptable, while optionally providing useful products from the purification treatment.

The present invention provides a process for the treatment of effluent, which comprises the steps of

(a) growing algae in a growth stage under aerobic conditions in a saline medium;

(b) treating a saline effluent containing organic degradable material with the algae in the presence of bacteria capable of digesting at least some of the organic material; and

(c) separating the liquid from the solid material.

In the first step of the process, the algae are grown under aerobic conditions in a saline medium. The saline medium used in step (a) may be any suitable water on which the algae will grow. Examples are defined media or enriched sea water media as formulated in the prior art, stationary water, such as tannery evaporation ponds, or the like on which the algae exists, or an aqueous system which has been specially obtained by treatment with algae in order to remove some undesired product from the water. Typical examples are waters containing halide ions which have been treated with halophilic algae, eg. of the species Dunaliella salina. Such starting materials may be any suitable industrial saline medium and includes effluents from the leather industry and/or saline organic wastes, eg. semi-solid organic wastes. Examples are effluents from tannery ponds, certain tannery and skin curers effluent, primary and secondary sewage sludges other tannery wastes and the like, which, if necessary have been treated with a brine to make them saline.

The algae for the growth stage may be obtained from a pond system or photobioreactor, or from any other suitable source. They can be supplied to the algae growth stage of the process, eg. a pond system or photobioreactor, or the first-mentioned same pond system / photobioreactor from which they are obtained is used for the growth stage. Growth takes place under aerobic conditions producing oxygen and utilising carbon dioxide. Periodically, the growth stage apparatus (eg. pond(s) and/or bioreactor(s) used in step (a) can be cleared substantially of their contents by passing a stream comprising water and the algal cells to the effluent treatment stage and clearing the ponds and/or photobioreactor.

The algae may be any suitable algae that can be grown under saline conditions in a first stage and that can degrade organic degradable material or contribute to its degradation in a second stage. The algae may be halophilic algae belonging to the genus Dunaliella or genus Spirulina. Conveniently they may be of the species Dunaliella salina. Examples of other halophilic species are Dunaliella bardawil, Dunaliella parva, Dunaliella tertiolecta, Dunaliella primolecta, Dunaliella peircei, Spirulina platensis, Spirulina maxima and the like. All of these are well-known but the presently preferred species are Dunaliella salina and Spirulina platensis. Dunaliella salina is found in South Africa in tannery ponds and on saline water impoundments. It can be separated therefrom by physical means, eg. by filtration, centrifugation, etc. They can grow prolifically in high saline effluent.

The effluent treated in step (b) of the process of the invention can be any suitable effluent. Examples of suitable effluents include industrial organic saline effluents, and organic sewage effluents, provided that the salinity thereof has been suitably adjusted, where necessary for it to be degraded by the algae. Conveniently, the effluent being treated is an effluent which has been made saline using fresh brine.

In principle the effluent may be any suitable contaminated water containing salts, eg. halide ions, provided it does not contain a material which is sufficiently toxic to the algae to kill the algae. Simple experimentation will indicate whether the aqueous effluent source is suitable. Salts which may be present in the effluents include, particularly, sodium chloride, as well as sulphates, phosphates, carbonates and further metal ions, eg. potassium and chromium.

"Tannery effluents" can be used. They are effluents from the leather industry. They are known to be strong pollutants containing high salinities, high chemical oxygen demands ("CODs"), sulphides and heavy metals. The tannery effluent may, for example, be effluent from a tannery pond (which, in the past, often has been evaporated, thereby releasing the water into the atmosphere while retaining the contaminants), rate tannery effluent, skin curing effluent, unhairing liquor saline hide soak liquor and curers brine effluent. Hide soak liquor is the most saline effluent stream produced from the soaking of salted hides. Tannery effluents may be pretreated by addition of a flocculent (eg. an aluminium salt such as the chloride or sulphate) and/or by submission to cross-flow mircofiltration (CFMF), eg. using an Explochem system. In a CFMF process, saline hide soak liquors, or the like tannery effluent are clarified using a diatomaceous earth precoat.

When the effluent is a tannery effluent addition of a flocculent and/or a CFMF pretreatment conveniently may be carried out. When the effluent is not a tannery effluent, such a pretreatment may still be carried out.

The effluent may be from ponds, eg. from a high rate oxidation pounding system (HROP system). Alternatively so-called "fresh water" from an HROP system or treated sewage or water from a feedlot waste can be utilized provided that it is saline. A substantial portion of suspended organic solids may be removed from the system before treatment with the algae.

Non-tannery effluents may be used. They include organic effluents such as sewage effluents, which can be used if they have been made saline. High organic content effluents, such as substantially solid organic wastes, for example primary and secondary sewage sludges from an activated sewage sludge treatment process, can be treated in accordance with the invention provided that they are saline. Thus a brine may be added if necessary. No other pretreatment of such sludges has been found to be necessary. In the past, the solid material from such sludges has often been disposed to landfill, to sea or directly to anaerobic digestion.

In an organic effluent (i.e. solid waste) - treating embodiment of the invention there is prolific growth of halophilic algae in the growth stage. They are preferably cultivated in a high saline effluent in that stage and then used to treat sewage and other organic matter, and to biodegrade the solid matter. Such an organic effluent need not be pre-treated. As the growth rate of the halophilic algae can be predicted, the scale of the sewage, or other organic matter, waste degradation/dispersal system can be calculated.

The algae not only contribute oxygen to the process (which they have in common with fresh water algal HROP systems) but also often release significant quantities of fixed carbon photosynthate, eg. free glycerol. This enables a more effective breakdown of refractory organics, examples of which are primary and secondary sewage sludge and activated sludge solids.

Another algae that can be used is an alga of genus Spirulina, especially Spirulina platensis.

The genus Spirulina is correctly classified as a cyanobacterium but is often referred to as an alga. Strictly speaking, it is a blue-green bacteria which is susceptible to light, eg. sunlight when it acts as a photosynthetic bacterium. Certain species of Spirulina have previously been grown in defined substantially pure mineral media (eg. Zarouk's medium) as well as having been grown on other non-saline wastes such as feed stocks, piggery wastes and liquids from which organics have to be removed. During their growth, they produce further amounts of the Spirulina organism.

We have found that members of the genus Spirulina can be grown on a saline alkaline medium with a view to purifying such media provided that the medium is not toxic to the cyanobacterium.

A particularly interesting embodiment of the invention is the treatment of sewage sludges. When sewage is treated, it is in the liquid phase with solids in solution and suspension. In a first step, the suspended phase is caused to be sedemented out, producing the primary sludge. The liquid phase is then treated further to purify it and can be passed to an oxidative treatment where microorganisms grow on it. The microorganisms can be removed from the solution as the solid phase to form a biomass or secondary sludge, often known as activated sludge with the production of a substantially purified liquid stream. In the present invention, if this secondary sludge is transferred to a saline medium, a substantial breakdown occurs due to osmotic effects releasing certain organics to solution and a reduced amount of residual cell matter which forms the refractory component of the sludge. The organics in solution may be removed by an oxidation process involving algal interaction with other microorganisms. Algae may be separated to yield useful products such as biomass, pigments, glycerol and fatty acids. Residual sludge can be transferred to an anaerobic digester, together with algal biomass and photosynthate. Given enhanced nutritional status anaerobic digestion can proceed substantially completely to leave very small amounts of solid waste products. The gaseous products of anaerobic digestion may be used, eg. methane to fuel the incineration of final sludges from the anaerobic digester and carbon dioxide returned to enhance photosynthetic productivity in the algal growth stage.

One particular embodiment of the invention provides the treatment of a saline effluent with a cyanobacterium of genus Spirulina to which the medium is not toxic, separating the refractory material, supplying it to an anaerobic digester for further treatment and, if desired, separating useful products from the grown cyanobacteria (ie the algae).

When using genus Spirulina algae, the salinity of the medium may vary within wide ranges, eg. from 0,1 to 3-M NaCl, more preferably about 0,5-M NaCl.

The alkalinity can be at a pH greater than 8 and more conveniently from about 9 to 10,5.

A useful product of the process of the invention is an increased amount of the Spirulina Cyanobacteria which can be used as a nutrient or pigment or from which extracts may be obtained.

The salinity of the effluent provides a selective factor whereby the algae component can be predictably maintained - unlike existing state-of-the-art fresh water algal HROP systems where successions and fluxes of algal populations are common and lead to less predictable process conditions. In addition the salinity causes a degree of physical breakdown or lysis of the cell biomass of which activated and secondary sludges are composed making them more available to degradation by the system.

In the case of genus Dunaliella the saline medium conveniently is equivalent to 1,5 to 5 M sodium chloride. The Dunaliella alga releases carbon in the form of glycerol during its growth, eg in HROP systems and the glycerol can play a useful part in the supplementation of carbon to nitrogen ratios and hence in the rate of degradation of organics and especially refractory organics. If desired, additional carbon can be provided to ponds where the effluent is treated with the algae, eg. by means of carbon dioxide and/or cycling a high carbon effluent into the ponds or the like. The algae also provide oxygen for the degradation. Refractory organics are difficult to degrade organic materials eg. chitins, Keratins, etc. and other slowly biodegradable organic matter. The algal biomass produced by the Dunaliella alga can itself contain up to 50 % of glycerol (calculated on a dry weight basis). Therefore, if the Dunaliella alga is harvested and recirculated, it can significantly enhance the rate of the process of the invention. With the present invention, substantial cell recovery is possible.

The algae are grown in a first step, eg. separately in a high salinity pond or series of ponds, and then mixed into a second pond, or series of ponds, containing the effluent. Alternatively, a single pond, or series of ponds, may be used for both the growth step for the algae and the treatment step for the effluent. Instead of ponds, tubular photobioreactors or the like can be used. A cascade of ponds may be used. The ponds are open to the air.

The bacteria which cause the degradation usually do not have to be added from a separate source but usually are already present in the aqueous effluent starting material, irrespective of its source. On the other hand, such bacteria may be added, if desired, eg. with the algae.

The process of the invention enables solid material in the effluent to go into solution where it is degraded by the biodegrading bacteria which thrive on effluents. Due to the halophilic algae leaking photosynthate (especially carbon) into the medium even normally refractory organic materials can be degraded.

The bacteria present attack the cell material in the aerobic ponds or other digesters. This cell material previously was an intractable organic material and the increased carbon to nitrogen ratio allows substantially improved bacterial activity to take place.

A predictable level of photosynthetic carbon is possible where the salinity and composition of the effluent is known as this enables an estimated algal population to be produced. This in turn provides reasonable confidence limits for the working of the process. A problem with prior fresh water algal HROP systems was that there was a lack of predictability. The amount of photosynthetic carbon produced in such a saline solution can be estimated on the basis of test results at 30 to 50 tons per hectare (HA) per year and an oxygen yield of 100 to 200 tons/hectare (HA)/year.

After treatment according to the process of the invention, the purified water can be separated from the solid material, comprising biomass and/or algae in any suitable manner, eg. by adsorption, flocculation, agglutination, filtration, particularly cross flow ultrafiltration (CFUF), CFMF, or the like. The solid material contains the cells together with useful material, eg. beta-carotene and glycerol if Dunaliella algae have been used. Addition of a flocculent, eg. an aluminium salt such as aluminium chloride or sulphate may take place before the separation of the cells from the liquid. Thereafter the beta-carotene and glycerol, if these products are present can be separated from the cells and purified if desired.

One particularly convenient method of separating the solid material from the purified water involves crossflow ultrafiltration, with or without the use of a flocculent and/or utilizing diafiltration. The use of a polysulfone coated tubular ultrafiltration system, eg. that know commercially by the name "Membratek" has proved very suitable. Any suitable flocculent may be used. We have found that aluminium flocculants, especially aluminium chloride and sulphate to be very suitable.

The separated algae can be treated further in order to produce useful products from them, such as Beta-carotene, glycerol etc. Such products can be obtained by stressing the treated cells in a stress unit followed by further cross-flow ultrafiltration (CFUF) and concentration of the cell concentrate. If aluminium has been used as a flocculent, it can be removed from the solids by means of an acidification in the cross-flow ultrafiltration step following stressing, eg. using hydrochloric acid to provide a permeate containing aluminium ions in solution. Treatment thereof with a base can lead to flocculation of the aluminium which then can be separated, eg. by sedimentation, filtration, centrifugation etc.

Cell concentrates of 10 to 15 % solids dry weight can be produced. Although some cell fracture may occur at higher solids recovery levels, desirable products frequently remain bound to the cells and do not pass through the filter with the permeate. In the case of Dunaliella cells, we have found that Beta-carotene remains bound to the cells up to a MW of 100 000 cut off. Cells with bound Beta-carotene do not pass through the filter, even if the cells are broken - since the cut off point of the filter for permitting the passing of particles is a MW of 100 000. Thus, effectively a two-step practical method has been provided, whereby substantially undamaged cells are separated in the CFUF.

With the invention, substantially a batch process effectively is provided. We have found that an improvement factor of about double is obtained with the batch process of the invention. Furthermore, the retention time in the growth stage can be improved. There is substantially no contamination of that stage as "clean" brine is supplied to it and the retention time is short. The "clean" brine can be recycled brine from the CFUF optionally mixed with fresh brine. The recycled brine is a substantially "clean medium so that contamination may effectively be avoided.

The permeate from the CFUF can be returned for the next growth cycle in a sterile condition to the growth stage thereby preventing the accumulation of contaminants and predators which are usually associated with continuous culture conditions.

The solid material which has been separated may be subjected to stressing, eg. by means of irradiation. This can be carried out in shallow ponds or narrow bore tubular photobioreactors subjected to an irradiation stress. The times of treatment can be up to about eight days, more conveniently four to six days depending on the residual nitrogen levels and the irradiation.

Since conditions of irradiation by sunlight and the cell's nutritional status may vary considerably, especially where an organic effluent growth medium is used, it is of practical value in the operation of the stress unit and the manipulation of combinations of stress conditions to be able to monitor the process of stress induction together with metabolite accumulation (where this is beta-carotene). Abscisic acid (ABA) is a plant hormone which is probably formed via the xanthophyll cycle (zeaxanthin, antheraxanthin and all-trans-violaxanthin) and neoxanthin. Its presence in Dunaliella salina and relationship to beta-carotene accumulation has been described by Cowan A.K. and Rose P.D., 1991 Plant Physiology 97:798-803. In the stressing its appearance is linked to the metabolic processes that occur in the stress unit leading to enhanced beta-carotene production. Thus, monitoring the amount of ABA and/or the epoxidation state of the xanthophyll cycle (zeaxanthin plus antheraxanthin plus all-trans violaxanthin) can give an indication of the measure of stress. In other words, the induction of stress condition(s) may be monitored and/or manipulated and controlled by observing changes in intracellular abscisic acid and components of the xanthophyll cycle. Alternatively, the manipulation of ABA levels and/or zeaxanthin, antheraxanthin and all-trans-violaxanthin levels and/or rate of synthesis could result in a further enhancement of carotenoid production.

Other stress methods, eg. depriving the cells of nitrogen and/or of phosphate and/or of sulphate, and/or of providing a high salinity (3 to 4 M NaCl) in the stress stage can be used. The use of a small diameter transparent tube or shallow pond for irradiation is presently preferred.

By adjusting the salinity, salinity stress may be caused to the aqueous mixture thereby leading to a substantial conversion of starch reserves to glycerol. The glycerol can be recovered during the subsequent extraction phase.

The invention is particularly convenient for algal cells from a high rate oxidation ponding system (HROP), whether this is carried out in selective high salinity ponds or in closed tubular photobioreactors to contain or avoid contamination. Conveniently, the aqueous liquid from such ponds or photobioreactors can be supplied to the growth ponds. High inoculum levels, eg. up to 10 % of the log cultures, can be seeded into such ponds using high nitrogen, low salinity media to optimise cell production. Desirably, confluent growth should be achieved as rapidly as possible, preferably in a period of less than 10 days and more conveniently about four to seven days.

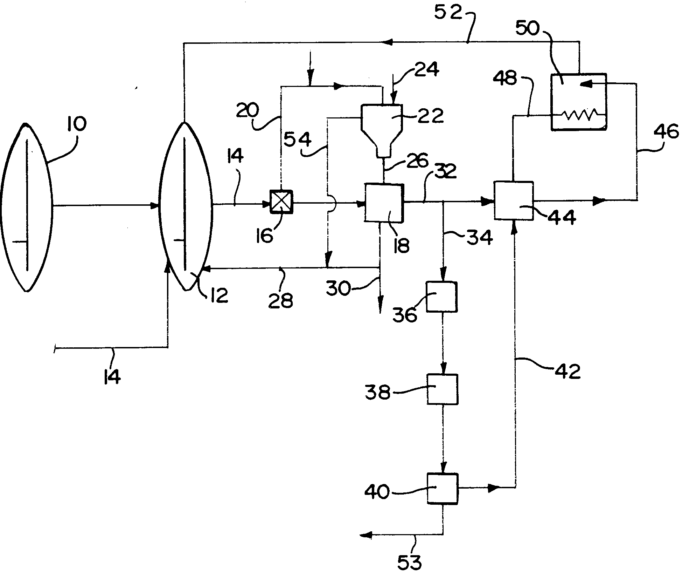

A schematic drawing illustrating the invention is set out on the attached figure.

In this figure, a growth pond 10 contains saline solution on which algae of genus Dunaliella are grown.

The algae are then supplied to pond 12. Alternatively, the algae can be grown in pond 12. The pond 12 further contains organic effluent received along path 14 and brine. Bacteria present in the effluent, and in the ponds 10 and 12, together with the effect of the algae and saline conditions, cause a portion of the organic material to pass into solution and be biodegradable.

If the effluent is a tannery effluent, an appropriate pretreatment step such as flocculation or CFMF, or both is carried out before the effluent reaches pond 12.

The material from the pond 12 then passes along line 14 to position 16 where it either passes directly to a cross-flow ultrafiltration (CFUF), - (alternatively CFMF where recovery of viable algae is not required) - step 18 or passes along line 20 to a flocculating apparatus 22 where aluminium is introduced along line 24 to cause flocculation. The flocculated material then passes along line 26 into the CFUF step 18. From here, liquid can be passed along line 28 back to the pond 12 or is removed from the system at 30. Alternatively, supernatant can pass directly from the flocculating apparatus 22 along 54 to 28. Solid material (and liquid as desired) passes along line 32. Thereafter it may pass along line 34 to a unit 36 where further CFUF and diafiltration takes place. The solid material is then stressed at 38 and desired products such as Beta-carotene and/or glycerol are removed at 40 in known manner.

The solids remaining can be disposed of directly along line 53 or alternatively passed along line 42 to an anaerobic digestion unit 44. Alternatively, the matter passing along 32 can lead directly to unit 44. The final solids are supplied from unit 44 along line 46, whereas gas formed by the anaerobic digestion passes along line 48 and is ignited in furnace 50. The final solids material can be burnt in the furnace 50. Carbon dioxide passing along line 52 back to pond 12 increases the carbon content in that pond.

This invention thus comprises a process which provides a functional use for saline water. Organic solids (eg. separated sewage sludges) may be added to water or organics may be already present in the water (eg. tannery effluents) which is then subjected to microbial oxidative treatment in the presence of photosynthetically produced oxygen from algae. This can considerably reduce the costs currently associated with the mechanical aeration of oxidative treatment systems with photosynthate enhancing microbial degradation rates. A predictable algal population can be produced given the selective factors of salinity and/or alkalinity, providing products of value and a functional use for saline water.

The following non-limiting Examples illustrate the invention:

Example 1

Pretreatment

Saline hide soak liquors (produced by soaking salt cured hides) were subjected to pre-clarification treatment by cross-flow microfiltration (CFMF) using an Explochem system and/or aluminium sulphate (or chloride) flocculation.

The CFMF treatment comprised clarifying the saline hide soak liquors using a diatomaceous earth precoat. The following presents typical results comparing the raw feed and clarified permeate.

HIDE SOAK LIQUOR - CFMF

|  | Feed | Clarified |
|---|---|---|
| Supernatant |  |  |
| Suspended Solids (mg/ml) | 1270 | 40 |
| Permanganate Value (mg/l) | 325 | 61 |
| Chemical Oxygen Demand (mg/l) | 3960 | 1020 |
| pH | 6.7 | 6.3 |
| Turbidity (NTU) | 370 | 95 |

The aluminium sulphate flocculation was carried as follows:

Effective dosages of flocculent were first determined in jar tests and a dose of 0,5 g/l was found to be effective with 50 % settling of solids completed in 1 hour. Liquor pH = 6,7.

### HIDE SOAK LIQUOR - ALUMINIUM FLOCCULATION

|  | Feed | Clarified |
|---|---|---|
| **Supernatant** | | |
| Suspended Solids (mg/ml) | 1270 | 265 |
| Permanganate Value (mg/l) | 325 | 106 |
| Chemical Oxygen Demand (mg/l) | 3960 | 1580 |
| pH | 6.7 | 6.6 |
| Turbidity (NTU) | 370 | 100 |

Growth step

A mixed culture of <u>Dunaliella salina</u> and heterotrophic bacteria previously adapted to grow in saline hide soak liquor was inoculated into a quantity of fresh hide soak liquor (diluted 1:1 with tap water and salinity adjusted to 1,5 M NaCl) and treated as indicated above to give counts of $0,5 \times 10^5 . ml^{-1}$ algae (calculated in a hemocytometer) and $40 \times 10^4 ml^{-1}$ bacteria (counted as colony forming units on 1,5 M NaCl plate count agar). The inoculated medium was incubated at 30°C and illuminated at 100 $uE.m^{-2}.sec^{-1}$. in glass bottles. The organic load in the hide soak liquor was determined as chemical oxygen (COD) in $mg.l^{-1}$ and permanganate value (PV) in $mg.l^{-1}$. Growth of <u>Dunaliella</u> was not impaired in this medium diluted over the range of 25 % to 75 % and adjusted to salinity 1,5 M NaCl.

Organic load reduction over a ten day period is approximately 80 % and algal numbers increased to $6 \times 10^6 . ml^{-1}$.

A co-culture similar to that described in part 1(a) above, containing <u>D. salina</u> at approximately $0,5 \times 10^5 . ml^{-1}$ was inoculated into a 25 % dilution of hide soak liquor (1,5 M NaCl) illuminated and grown in an illuminated microprocessor controlled 51 New Brunswick Bioflow III fermentation system. Results typically show an organic load reduction of 5 - 15 % per day measured as $mg.l^{-1}$ COD.

A similar co-culture system to that described in part 1(b) above was set up as a continuous system, fed daily with aliquots of a 25 % dilution of hide soak liquor (salinity adjusted to 1,5 M NaCl). The cells produced were separated from the liquid.

The system typically achieves steady state when fed at between 5 - 8 % of culture volume per day.

The supernatant medium was adjusted to 1,5 M NaCl and pH 9.0, inoculated with <u>Dunaliella salina</u> and incubated in flasks in a controlled environment and in outdoor $1m^2$ stirred ponds. Algal productivity was determined by measuring $NaH[^{14}C]O_3$ fixation rates: Optimal rates of 20 mg. C. L. $M^{-2}.d^{-1}$ were achieved.

Example 2

Cells produced according to Example 1 were concentrated approximately 100 fold ($0.2 \times 10^5$ cells/ml to $25 \times 10^6$ cells/ml) by CFUF yielding a concentrate of approximately 1,5 % solids. The filter did not block and flux rates typically vary around 50 1. $m^{-2}.hr^{-1}$.

The filter concentrate was rediluted to starting cell concentrations in the growth medium, and the growth rate was measured and compared to control cultures not subjected to CFUF concentration. The growth rate of cells subsequent to treatment was not impaired by the CFUF concentration process.

Cells were stressed subsequent to CFUF treatment in 3 - 4 M NaCl and beta-carotene accumulation measured in cells exposed to light intensity of 100 $uE.m^{-2}.sec^{-1}$ compared with untreated controls. The rate of beta-carotene accumulation was not affected by the CFUF concentration treatment process.

The permeate produced by CFUF was free from bacteria in all cases (measured as colony forming units on 1,5 M NaCl plate count agar) which enables the recycle of "clean" medium free of infectious particles.

Example 3

Algal growth on secondary sewage sludge

Secondary sludge was collected from an activated sludge system treating municipal effluent. Solids were removed by centrifugation and washed 3 x in tap water to remove dissolved organics. The washed sludge was

7

resuspended in a saline (3,5 M NaCl) medium at a concentration of 686 mg. $1^{-1}$. After 48 hours the supernatant was removed and the permanganate value (PV) determined. Supernatant contained 157 mg. L-1 finely dispersed suspended and dissolved solids or 23 % of supernatant PV. Dissolved solids accounted for 50 mg. $L^{-1}$ or 8.6 % of supernatant PV.

The supernatant medium was adjusted to 0.5 M NaCl and pH 10.5, inoculated with <u>Spirulina platensis</u> and incubated in flasks in a controlled environment and in outdoor 1 $M^2$ stirred ponds. <u>Spirulina platensis</u> productivity was determined by measuring Na H$[^4C]O_3$ fixation rates. Average optimal rates of 15 mg $C.M^{-2}.d^{-1}$ were achieved.

In the case of <u>D. salina</u>, the supernatant medium was adjusted to 1,5 M NaCl and pH 9, and incubated under the conditions described above. Fixation rates of 12 mg $C.M^{-2} .d^{-1}$ were recorded.

## Example 4

### Spirulina growth in tannery evaporation ponds

Growth rates of <u>Spirulina platensis</u> in media derived from tannery evaporation/facultative ponds was measured by NaH$[^4C]O_3$ fixation.

A typical analysis of the ponds liquors is as follows:

```
PV                296        mg/1
TDS             16700        mg/1
TDIS            11480        mg/1
SS                106        mg/1
TFN               114        mg/1
NH₃                37        mg/1


Nitrate            25        mg/1
Sulphate         1875        mg/1
pH                9.3
```

Where C fixation was measured in situ using the productivity bottle method rates of 4-10 g $C.M^2d^{-1}$ were recorded. Where this medium was placed in 1 $m^2$ outdoor High Rate Oxidation Ponds and inoculated with <u>Spirulina platensis</u> at a biomass rate of 0.36 mg chlorophyll a. $M^{-2}$, optimal productivities of 25 g L. $M^{-2}.d^{-1}$ were achieved. This was accompanied by an organic load reduction of 90 - 95 % COD with hydraulic retention times of 4 - 9 days.

## Example 5

### Spirulina growth in unhairing liquor

Unhairing liquor is a highly alkaline, high COD medium produced as a preliminary step in treating hides. Solids can be separated by settling and the following presents a typical analysis of the resulting supernatant medium.

| | |
|---|---|
| pH | 12.1 |
| conduct | 1025 $m^3.M^{-1}$ |
| PV | 6830 mg/l |
| COD | 26000 mg/l |
| TDS | 1,22 % |
| TDIS | 0,44 % |
| SS | 823 mg/l |
| SEH Solids | 6 mg/l |
| sulphide | 557 mg/l |
| sulphate | 220 mg/l |
| TKN | 885 mg/l |
| Ammonia | 196 mg/l |
| Calcium | 180 mg/l |
| Alkalinity (methyl orange) | 700 mg/l |

Adapted Spirulina platensis cultures were inoculated into flasks of various dilutions of this effluent and salinity adjusted to 0.5 M NaCl and pH to 10.5. Flasks were incubated in a constant environment chamber-temperature of 30°C and illumination 100μmol. $M^{-2}.sec^{-1}$.

Outdoor 1 $m^2$ High Rate Oxidation Pond studies showed Spirulina productivity of 10 mg L. $M^{-2}.d^{-1}$ and organic load reductions of 80 - 90 % where the concentrated effluent was fed at a rate of 5 % $d^{-1}$.

## Claims

1. A process for the treatment of effluent which comprises the steps of;
    growing algae in a growth stage under aerobic conditions in a saline medium;
    treating a saline effluent containing organic degradable material with the algae in the presence of bacteria capable of digesting at least some of the organic material; and
    separating the liquid from the solid material.

2. A process as claimed in claim 1, wherein the effluent is a tannery effluent.

3. A process as claimed in claim 2, wherein the effluent is raw tannery effluent, skin curing effluent or saline hide soak liquor.

4. A process as claimed in claim 2 or claim 3, wherein the tannery effluent has had a flocculent added to it and/or has been submitted to cross-flow microfiltration.

5. A process as claimed in claim 1, wherein the effluent is an organic sewage effluent.

6. A process as claimed in claim 5, wherein the effluent is a sewage sludge.

7. A process as claimed in any of the preceding claims, wherein the algae are of the genus Dunaliella.

8. A process as claimed in any of the preceding claims wherein the algae are Dunaliella salina.

9. A process as claimed in any of the preceding claims wherein the saline content in the growth stage is 1,5 to 5 M NaCl.

10. A process as claimed in any of claims 1 to 6, wherein the algae are of the genus Spirulina.

11. A process as claimed in claim 10, wherein the algae are Spirulina platensis.

12. A process as claimed in any of the preceding claims, wherein the separation of the liquid from the solid material involves cross-flow ultrafiltration or cross-flow microfiltration.

13. A process as claimed in any of claims 1 to 9, wherein the separated solid material is subjected to stress.

14. A process as claimed in claim 13, wherein the separated solid material is subjected to irradiation, high salinity and/or is deprived of nitrogen and/or of phosphate and/or of sulphate.

9

15. A process as claimed in claim 14, wherein cross-flow ultrafiltration is carried out after the stressing.

16. A process as claimed in claim 10 or claim 11, wherein the biomass containing increased amount of the Spirulina algae is subjected to a separation of the algae from the remainder.

17. A process as claimed in claim 4, wherein the separated liquid containing flocculant is subjected to cross-flow ultrafiltration under acidic conditions, followed by treatment with a base to remove the flocculant.

18. A process as claimed in claim 1, substantially as described with reference to Examples 1 or 2.

19. A process as claimed in claim 1, substantially as described with reference to any of Examples 3 to 5.

20. A new process for the treatment of effluent, substantially as described with reference to the accompanying drawing.

**European Patent Office**

## EUROPEAN SEARCH REPORT

Application Number

EP 92 30 6078

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | CHEMICAL ABSTRACTS, vol. 101, no. 14, 1 October 1984, Columbus, Ohio, US; abstract no. 116211c, & CHAUDHARI,P.R. ET AL.-INDIAN J.ENVIRON.HEALTH vol. 25, no. 4, 1983, pages 275 - 281 | 1,5,6, 10,11 | C02F3/32 C02F9/00 |
| A | DATABASE WPIL Section Ch, Week 8548, Derwent Publications Ltd., London, GB; Class D15, AN 85-299526Q & JP-A-60 206 496 (HASHIMOTO S.) 18 October 1985 * abstract * | 1,5,10 | |
| A | AU-A-486 999 (VITAMINS(AUSTRALIA) PTY.LTD.) 19 February 1976 * page 3, paragraph 5 * * page 4, line 30 - page 5, line 8 * * claim 1 * | 1,4,7,8, 16,17 | |
| A | US-A-4 209 388 (DEFRAITES, A.A.) 24 June 1980 * column 2, line 30 - line 34 * * claim 1 * | 1,5 | TECHNICAL FIELDS SEARCHED (Int. Cl.5 ) C02F |

The present search report has been drawn up for all claims

| Place of search | Date of completion of the search | Examiner |
|---|---|---|
| THE HAGUE | 16 OCTOBER 1992 | GONZALEZ ARIAS,M.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)